# EUROPEAN PATENT SPECIFICATION

(11) **EP 0 535 148 B1**
(45) Date of publication and mention of the grant of the patent: **18.03.1998**
(21) Application number: 91913153.2
(22) Date of filing: 10.06.1991
(51) Int. Cl.: A61K 38/18, A61K 38/20, C12N 5/00

(54) **STIMULATION OF BONE MARROW STROMAL AND PROGENITOR CELLS**
STIMULIERUNG DER STROMA- UND VORLÄUFERZELLEN IM KNOCHENMARK
STIMULATION DE CELLULES PARENTES ET STROMALES DE MOELLE OSSEUSE

(30) Priority: 08.06.1990 US 536108
(43) Date of publication of application: 07.04.1993
(73) Proprietor: NEW YORK UNIVERSITY, New York, NY 10016 (US); MEMORIAL SLOAN-KETTERING CANCER CENTER, New York, NY 10021 (US)
(72) Inventor: WILSON, E., Lynette, New York, NY 10021 (US); RIFKIN, Daniel, B., New York, NY 10021 (US); GABRILOVE, Janice, New York, NY 10021 (US)
(74) Representative: VOSSIUS & PARTNER
(86) International application number: US9104146
(87) International publication number: WO9118620

(56) References cited:
- WO-A-89/00198
- WO-A-92/11355
- US-A- 4 785 079
- FASEB JOURNAL vol. 3, no. 3, 9 February 1989, BETHESDA, MD US page A523 MIYAGAWA, Y. ET AL. 'Fibroblast growth factor (FGF)-facilitated immature hemopoietic cell lines'
- PROCEEDINGS OF THE NATIONAL ACADEMY OF SCIENCES OF USA vol. 85, June 1988, WASHINGTON US pages 4360 - 4364 SONODA, Y. ET AL. 'Analysis in serum-free culture of the targets of recombinant human hemopoietic growth factors: interleukin 3 and granulocyte/macrophage-colony-stimulating factor are specific for early developmental stages'
- GROWTH FACTORS vol. 3, no. 3, August 1990, pages 231 - 236 OLIVER, J. ET AL. 'Long-term culture of human bone marrow stromal cells in the presence of basic fibroblast growth factor'
- Exp. Hematol., Volume 18, No. 4, issued May 1990, Wang et al., "Disecting the Hematopoietic Microenvironment. VI. The Effects of Several Growth Factors on the in vitro Growth of Murine Bone Marraw CFU-F", pages 341-347, see the Abstract.
- Faseb J., Volume 1, issued 1987, THOMAS, "Fibroblast growth factors", pages 431-440, see the paragraph bridging pages 437 and 438.
- "Illustrated Stedman's Medical Dictionary", 24th ed., published 1982 by Williams & Wilkins, see page 945, "Neuroectoderm".

## Description

The present invention in the field of cell biology and medicine relates to the use of fibroblast growth factors and particularly basic fibroblast growth factor for the stimulation of bone marrow cells in vitro and in vivo.

### Bone Marrow Cells: Culture and Stimulation

Tissue culture techniques have been developed that permit the long term maintenance of hemopoietic cells in vitro (Dexter, T.M., Acta Haemat. 62:299-305 (1979)). A vital component of this culture system is the prior formation of a bone marrow-derived adherent stromal cell layer in the culture vessel. The stromal layer consists of endothelial. cells, fibroblasts, adipocytes, and macrophages (Weiss, L., J. Morphol. 117:467-538 (1965); Lichtman, M.A., Exp. Hematol. 9:391-410 (1981)). Different culture conditions enable selective proliferation of cells of the B lymphocyte (Whitlock, C.A. et al., J. Immunol. Methods 67:353-369 (1984) or myeloid (Dexter et al., J. Cell. Physiol. 91:335-344 (1977)) cell lineage in stromal cell-dependent long term cultures. Certain cytokines such as IL1 and IFN-gamma have been shown to increase the production of various factors by adherent cells which may promote cell proliferation or differentiation (Zucali, J.R. et al., J. Clin. Invest. 77:1857-1863 (1986) ; Philip, R. et al., Nature 323:86-89 (1986)). Little is known about the effects of other lymphokines on human bone marrow stromal cells, partly because it has been difficult to obtain large quantities of these cells.

Hemopoietic stem cells are a class of cells, largely limited to the bone marrow, which have been defined functionally by the characteristics of extended self-renewal and the capacity to mature into one or more differentiated forms of blood cells, such as granulocytes, monocyte/macrophages, lymphocytes, erythrocytes, and megakaryocytes (which give rise to platelets). In vitro, stem cells appear to have a limited proliferative capacity, and are not able to divide indefinitely.

Hemopoietic cells appear to be intrinsically incapable of unstimulated cell division. The granulocytemacrophage colony-stimulating factors (GM-CSF, G-CSF, M-CSF) are specific glycoproteins that regulate the production, differentiation, and function of two related leucocyte populations in the blood, the granulocytes and the monocytes/macrophages.

Bone marrow transplantation is an increasingly common form of therapy for a number of diseases which involve dysfunction of hemopoietic cells (for example, aplastic anemia), or which include treatments which irreversibly damage hemopoietic cells (for example chemotherapy and radiotherapy of cancer). Due to clinical problems related to imperfect matching of donors and recipients, autologous bone marrow transplantation, where the patient serves as his own donor, is preferable when possible.

Autologous bone marrow transplantation involves removal of less than 5% of the total content of the recipient bone marrow, which is stored frozen and reinfused when appropriate. Mauch et al. (Blood 74:872-825 (1989)), studying animal transplant models, found a decrease in stem cell content and self-renewal capacity of bone marrow in transplant recipients that was proportional to the dose of transplanted bone marrow cells. This decrease (not reflected in peripheral blood leucocyte counts or bone marrow cellularity) was observed after initial bone marrow recovery, and did not change with time after transplantation, demonstrating a permanent loss in bone marrow self-renewal capacity.

Myelosuppression (a suppression in the generation of leucocytes, especially granulocytes or neutrophils) is a common and serious complication of cancer therapy because most chemotherapeutic agents lack specificity for malignant cells, and bone marrow stem cells and other immature blood cells which must proliferate are particularly susceptible to damage from chemotherapeutic drugs and radiation. A major cause of treatment-related deaths in cancer patients is infection, which is a function of both the duration and the severity of neutropenia. The use of autologous bone marrow transplantation has allowed more intensive, and thus effective, chemo- and radiotherapy for a variety of neoplasms. However, morbidity and mortality are high during the nearly three-week period required for the autologous bone marrow to successfully engraft and begin hemopoietic reconstitution.

Myelosuppression can also occur following administration of antibiotics, antidepressants, nonsteroidal antiinflammatory drugs, anti-viral agents, or following treatment of thyroid disease. Myelosuppression also accompanies a variety of immunosuppressive disorders, such as AIDS.

Brandt et al. (New Eng. J. Med. 318:869-876 (1988)), describe the use of recombinant human granulocyte-macrophage colony-stimulating factor (GM-CSF) for hemopoietic reconstitution after high-dose chemotherapy and autologous bone marrow transplantation and found accelerated myeloid recovery over a range of tolerable GM-CSF doses.

### Fibroblast Growth Factors

Many peptides with potent stimulatory effects on proliferation of either epithelial or mesenchymal cells have been identified. Because of their regulatory action on tissue growth, these peptides have been collectively termed "growth factors." Unfortunately, most growth factors were named according to the biological activity assayed during their original identification and isolation. However, it is now becoming evident that many growth factors have a much wider range of action than the biological activity for which they were originally named, and most are now considered to be multifunctional. Furthermore, many disparately named factors have, upon purification, turned out to be a single molecular entity.

Basic and acidic fibroblast growth factors (bFGF and aFGF) are peptides having an apparent molecular weight (Mr) of about 16 - 25 kilodaltons which act as potent mitogens and differentiation factors for a wide variety of cells derived from the embryonic mesoderm and neuroectoderm. Basic FGF (pI 9.6) was first identified by its ability to induce proliferation and phenotypic transformation of mouse fibroblasts (BALB/c 3T3 cells) (Gospodarowicz, D. Nature 249:123-127 (1974)). bFGF is multifunctional; it can stimulate proliferation and induce or delay differentiation. Basic FGF stimulates other critical processes in cell function as well, though the mechanisms of FGF's various actions have yet to be clarified (Rifkin, D.B. et al., J. Cell Biology 109:1-6 (1989)).

Basic FGF has been purified from most mesodermor neuroectoderm-derived normal or malignant tissues or cells that share a common property: strong angiogenic potential, that is, an ability to form new blood vessels. Structural studies have shown that bFGF is a single chain peptide composed of 155 amino acids, which can also exist in a truncated form lacking the first 15 N-terminal amino acids. Truncated bFGF is as potent as native bFGF in binding to cell surface receptors and in biological assays, indicating that the N-terminal region of bFGF is not necessary for binding or bioactivity. Depending upon the tissue source, either intact or truncated forms of bFGF are found. It is not known whether the truncated form coexists with the whole molecule in the tissues, or is an artifact of proteolytic cleavage during the extraction and isolation process.

Although human bFGF cDNA and genomic clones predict a 17.8 kDa gene product of 155 amino acids, bFGF protein isolated from human placenta contains two additional amino acids N-terminal to the predicted initiator methionine. The human cell line SK-HEP-1 was found to coexpress 4 molecular forms of bFGF (17.8, 22.5, 23.1, and 24.2 kDa). The 17.8 kDa bFGF protein is translationally initiated at the previously predicted methionine (AUG) codon, whereas, the 22.5-, 23.1-, and 24.2-kDa proteins initiate at unusual non-AUG codons. The higher molecular weight forms are colinear N-terminal extensions of the approximately 18 kDa bFGF. (See, for example, Florkiewicz et al., Proc. Natl. Acad. Sci. USA 86: 3978-3981 (1989)).

Many organs that contain bFGF are heavily vascularized. Vascular endothelial cells express the bFGF gene and synthesize bioactive bFGF. bFGF is also expressed in a wide variety of normal diploid cells (all of which are sensitive to bFGF in vitro) and in various tumors derived from those cells.

Cell types that respond to bFGF bear highly specific FGF cell surface receptors. Neither bFGF nor aFGF binds to receptors for other growth factors, and other growth factors do not bind to the FGF receptors. Studies of cross-linking of bFGF or aFGF to their receptors (on BHK-21 cells) indicated that both factors interact with the same two membrane components having an Mr of 145 and 125 kD, which apparently differ only by their degree of glycosylation.

Interaction of bFGF with a cell leads to increased membrane fluidity and ruffling, which correlate with rapid changes in the dynamic structure of the actin cytoskeleton. Basic FGF also stimulates the synthesis of various specific proteins, especially secreted proteins. One of these secreted proteins, the "major secreted protein," has recently been shown to be a thiol-dependent cathepsin. FGF also stimulates synthesis and release of a glycoprotein originally named "mitogen regulated protein," which is a member of the prolactin/growth hormone family and is identical to proliferin.

Basic FGF can act as a "competence" factor for normal diploid cells, wherein brief exposure induces a "commitment" to cell division. Progression through the cell cycle is further mediated by plasma constituents including transferrin and high density lipoproteins.

Basic FGF has both acute and long-term effects on the morphology and growth pattern of responsive cells; bFGF increases migratory activity and causes confluent cultures of 3T3 cells to appear "transformed" due to reduced cell-substratum adhesion, growth in crisscross pattern, and increased membrane ruffling. Basic FGF can also induce the growth in soft agar of nontransformed cells and can potentiate the effect of transforming growth factor-β (TGFβ). Basic FGF is a potent mitogen for mesoderm-derived cells, triggering cell proliferation with half-maximal and maximal effects at 1.5 and 10 pM, respectively. Basic FGF is mitogenic both for cells seeded at extremely low densities (for example under cloning conditions) and for low density cultures, and greatly reduces the average cellular doubling time. This is primarily due to a shortening of the G1 phase of the cell cycle.

Basic FGF stabilizes the phenotypic expression of cultured cells. This property is particularly interesting, since it has enabled long-term culturing of cell types that otherwise would lose their normal phenotype in culture when passaged repeatedly at low density. This biological effect of bFGF has been studied in detail in endothelial cells derived from large blood vessels or cornea that were cloned and maintained in the presence of bFGF and then deprived of it for various intervals. Basic FGF can induce capillary endothelial cells to invade a three-dimensional collagen matrix and to organize themselves to form characteristic tubules that resemble blood capillaries. Concomitantly, bFGF stimulates endothelial cells to produce a urokinase-type plasminogen activator, a protease that has been implicated in the neovascular response. Thus, bFGF can stimulate processes that are characteristic of angiogenesis in vivo, including endothelial cell migration, invasion, and production of plasminogen activator.

Basic FGF also acts as a differentiation factor for various cells, including chondrocytes, sheep preadipocytes, fibroblasts, astrocytes, and oligodendrocytes; bFGF treatment of neural cells induces both neurite outgrowth and ornithine decarboxylase activity. Not all of the effects of bFGF on cell differentiation are stimulatory. For example, bFGF can delay differentiation and fusion of normal diploid myoblasts. In some established myoblast cell lines, bFGF and aFGF can decrease creatine phosphokinase expression.

FGF also significantly delays the ultimate senescence of cultured cells, such as that of granulosa cells, adrenal cortex cells, lens epithelial cells, and vascular endothelial cells. When maintained in the absence of bFGF, these cells have a limited life-span, whereas in its presence, they can proliferate for more than 200 generations. Developmentally, bFGF promotes limb regeneration in lower vertebrates, and may play a role in the early development of the nervous system. Basic FGF promotes both the survival and differentiation of nerve cells derived either from the hippocampus or the cortex. FGF may also have pronounced effects on the proliferation and differentiation of astrocytes and oligodendrocytes by influencing their glial properties during normal development or subsequent to a specific pathogenic event.

Basic FGF differs from other growth factors such as epidermal growth factor (EGF), platelet derived growth factor (PDGF), or TGFβ, by its ability to stimulate proliferation of all of the cell types involved in wound healing, in vitro as well as in vivo. These cell types include capillary endothelial cells, vascular smooth muscle cells, fibroblasts, chondrocytes, and myoblasts. Basic FGF also increases the formation of granulation tissue in vivo, the synthetic function of fibroblasts and myoblasts, rate of re-epithelialization of detached epidermis and chondrossification. Basic FGF acts as a potent angiogenic factor in vivo and is the major angiogenic agent in healthy vascularized tissues such as corpus luteum, adrenal gland, kidney, and retina.

Due to the widespread distribution of bFGF, and its broad range of target cells, at least 23 different names have been given to the molecule known as bFGF (leukemic growth factor (thymus), macrophage growth factor, embryonic kidney-derived angiogenesis factor 2, prostatic growth factor, astroglial growth factor 2, endothelial growth factor, tumor angiogenesis factor, hepatoma growth factor, chondrosarcoma growth factor, cartilage-derived growth factor 1, eye-derived growth factor 1, heparin-binding growth factors class II, myogenic growth factor, human placenta purified factor, uterine-derived growth factor, embryonic carcinoma-derived growth factor, human pituitary growth factor, pituitary-derived chondrocyte growth factor, adipocyte growth factor, prostatic osteoblastic factor, mammary tumor-derived growth factor, glial growth factor, and brain-derived neurotropic factor).

Basic FGF is unique among growth factors in its intracellular localization and lack of a signal sequence generally required for secretion, which raises questions concerning how and when bFGF is released.

FGF has been used for the treatment of ischemic heart disease (U.S. Patents Nos. 4,296,100 and 4,378,347 to Franco) where it was found to increase blood flow in the heart for sustained periods of time after myocardial infarction.

Nevo et al. (U.S. Patent No. 4,642,120) disclose the use of FGF for repairing defects of cartilage and bones.

Senoo et al. (European Patent Publication EP281822) disclose a mutein of bFGF which can be used to accelerate cell growth in vitro and can act as a healing accelerator for burns and a therapeutic drug for thrombosis.

Arakawa et al. (European Patent Publication EP320148) disclose recombinant bFGF analogs which possess at least one of the biological properties of mammalian bFGF factor. The bFGF appears to induce neovascularization, re-epithelization and wound repair.

It is an object of the present invention to overcome the aforementioned deficiencies in the prior art.

It is another object of the present invention to culture large number of progenitor cells for use in bone marrow transplantation by stimulating proliferation of hemopoietic progenitor cells with a fibroblast growth factor, preferably basic fibroblast growth factor (bFGF), or a functional derivative thereof.

It is yet another object of the present invention to treat hemopoietic progenitor cells in vitro with a proliferating effective amount of a FGF or a functional derivative thereof for a time sufficient to permit proliferation of the hematopoietic progenitor cells to increase their numbers for more effective bone marrow transplantation.

It is a further object of the present invention to accelerate establishment of the bone marrow graft and reconstitution of hemopoietic processes. This is achieved by bFGF or a functional derivative thereof, alone or in combination with one or more colony stimulating factors, which are for administration to a bone marrow transplant recipient.

This combination therapy is particularly useful for regulating blood cell production and treating disease states caused by hemopoietic dysfunction (such as aplastic anemia or neutropenia) or hyperplasia (such as various forms of leukemia).

It is still another object of the present invention to treat certain marrow aplastic or dysplastic conditions by stimulating endogenous cytokines using bFGF or a functional derivative thereof.

It is another object of the present invention to increase the concentration of progenitor cells in a bone marrow donor prior to transplantation, in order to increase the effectiveness of the transplanted bone marrow, by administering bFGF or a functional derivative thereof.

The addition of very small quantities (on the order of nanogram/ml) of FGF to cultures of hemopoietic progenitor cells greatly increases the rate of proliferation of these cells as well as the final cell density attained. This technique is therefore useful for providing large numbers of human bone marrow stromal cells that can be used to maintain cultures of hemopoietic cells for therapeutic and research purposes.

Thus, the present invention is directed to the use of a fibroblast growth factor or a functional derivative thereof for the preparation of a composition for stimulating proliferation of hemopoietic progenitor cells in vivo or in vitro.

It also refers to a method for proliferating hemopoietic progenitor cells cultured in vitro, comprising culturing said hemopoietic progenitor cells in a culture medium containing a proliferating effective amount of a fibroblast growth factor or functional derivative thereof, for a time sufficient to permit proliferation of the hematopoietic progenitor cells.

The present invention further encompasses a pharmaceutical composition for accelerating the engraftment of transplanted hemopoietic bone marrow cells in a mammalian subject or for treating a mammalian subject having a disease associated with dysplasia or aplasia of a bone marrow cell lineage comprising a combination of a fibroblast growth factor or a functional derivative thereof and at least one colony stimulating factor.

The invention is also directed to the use of a fibroblast growth factor or a functional derivative thereof for the preparation of a pharmaceutical composition for stimulating the proliferation of hemopoietic progenitor cells in the bone marrow of a prospective mammalian bone marrow donor in vivo, said pharmaceutical composition being suitable for administration at a time prior to the harvesting of said bone marrow or of a pharmaceutical composition for treating a mammalian subject having a disease associated with dysplasia or aplasia of a bone marrow cell lineage.

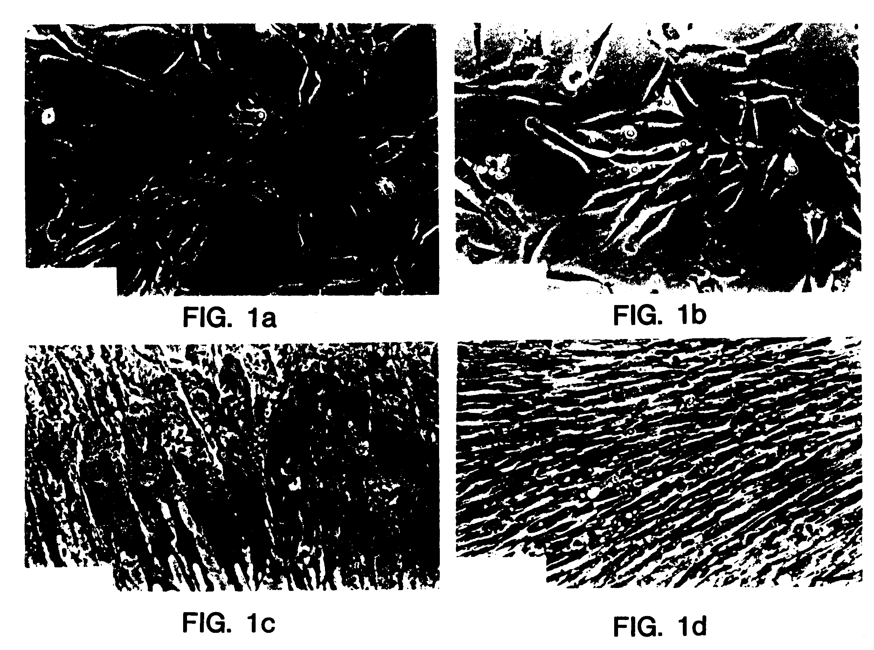

**Figure 1** is a set of photomicrographs showing the effect of bFGF on the morphology of bone marrow (BM) stromal cells. Phase constant micrographs of stromal cultures at different densities in the absence (a) and (c) and in the presence (b) and (d) of 20 ng/ml of bFGF. The scale bar = 50 µm.

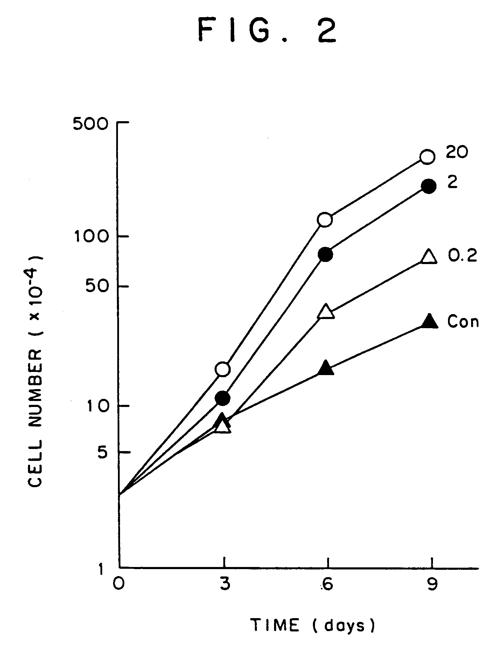

**Figure 2** is a graph showing the effect of various concentrations of bFGF on bone marrow stromal cell growth. Bone marrow stromal cells that had been passaged 3 times were seeded in stromal medium at 3 x 10⁴ cells per 35 mm culture dish in the absence (▲) or presence of bFGF at 20 (○), 2(●), or 0.2 (△) ng/ml bFGF. Duplicate dishes were removed and the cell numbers determined at the indicated times.

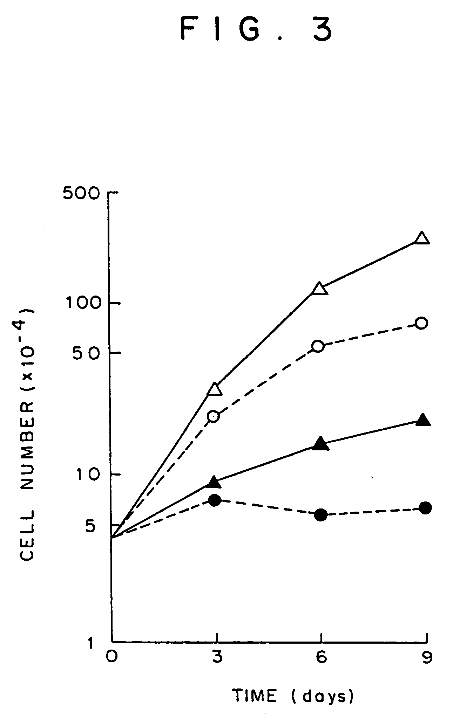

**Figure 3** is a graph showing the effect of bFGF on growth of bone marrow stromal cells in different media. Bone marrow stromal cells that had been passaged twice were seeded at 6 x 10⁴ cells per 35 mm dish in stromal medium (▲―▲), stromal medium containing 20 ng/ml bFGF (△―△), RPMI-1640 (with 10% FCS) (●--●) or RPMI-1640 (with 10% FCS) + 20 ng/ml bFGF (○--○). Duplicate dishes were removed and cell numbers were determined at the indicated times.

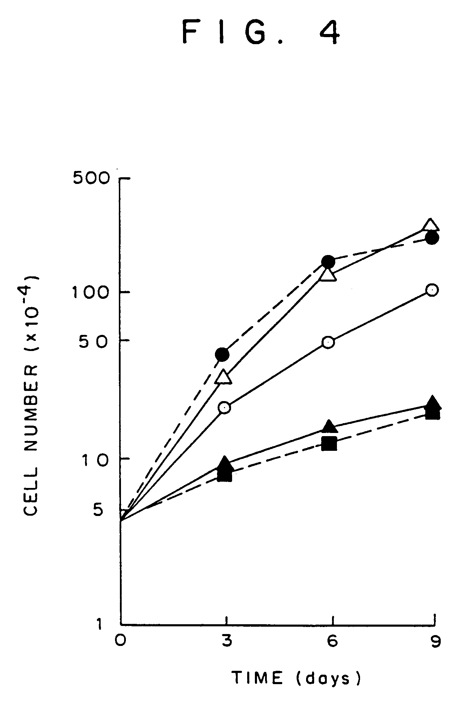

**Figure 4** is a graph showing the potentiation of the effects of bFGF on stromal cell growth by heparin. Bone marrow stromal cells that had been passaged twice were seeded at 6 x 10⁴ cells per 35 mm dish in stromal medium alone (▲―▲); stromal medium containing 2 ng/ml bFGF (○―○); 20 ng/ml bFGF (△―△); 2 ng/ml bFGF and 20 µg/ml heparin (●--●); and 20 µg/ml heparin alone (■--■). Duplicate dishes were removed and the cell numbers were determined at the indicated times.

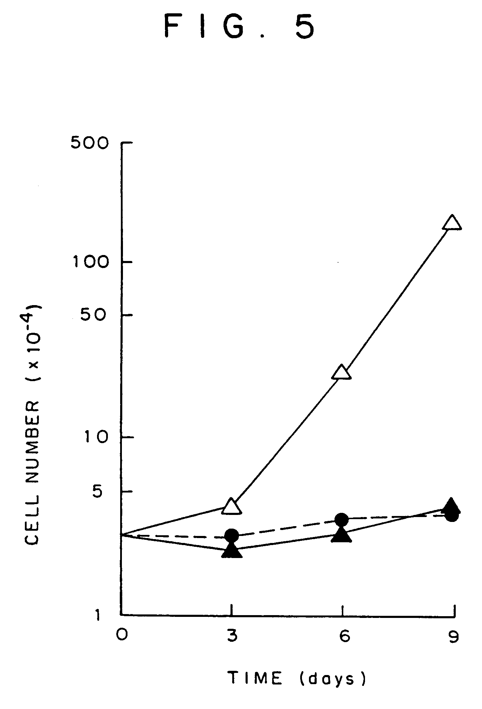

**Figure 5** is a graph showing the reversibility of the effects of bFGF on cell growth. Bone marrow stromal cells that had been passaged 4 times were used. bFGF at 20 ng/ml was added to some cultures which were passaged twice in the presence of bFGF over a period of 5 weeks. These cultures were then trypsinized and seeded at 3 x 10⁴ cells per 35 mm dish either in the continued presence of 20 ng/ml bFGF (△―△) or following its removal prior to trypsinization (●--●). Companion cultures that had been passaged 4 times without bFGF were seeded at the same density (▲―▲). Duplicate dishes were removed, and the cell numbers were determined at the indicated times.

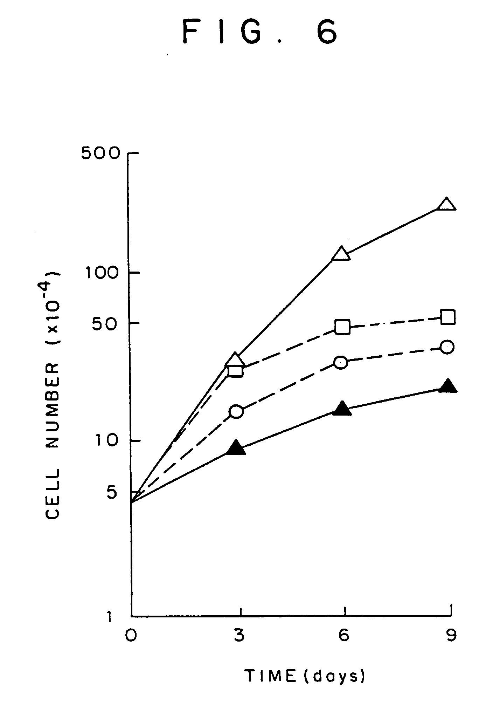

**Figure 6** is a graph showing the effects of brief exposure to bFGF on bone marrow stromal cell growth. Bone marrow stromal cells that had been passaged twice were seeded at 6 x 10⁴ cells per 35 mm dish. Medium alone (▲―▲) or medium containing 20 ng/ml bFGF was added to sets of culture dishes for 4 hr (○--○), 24 hr (□--□), or continuously (△―△). Following removal of bFGF-containing medium, the cultures were washed 3 times with 2 ml of stromal medium, and stromal medium alone was then added to these dishes. Duplicate dishes were removed and cell numbers were determined at the indicated times.

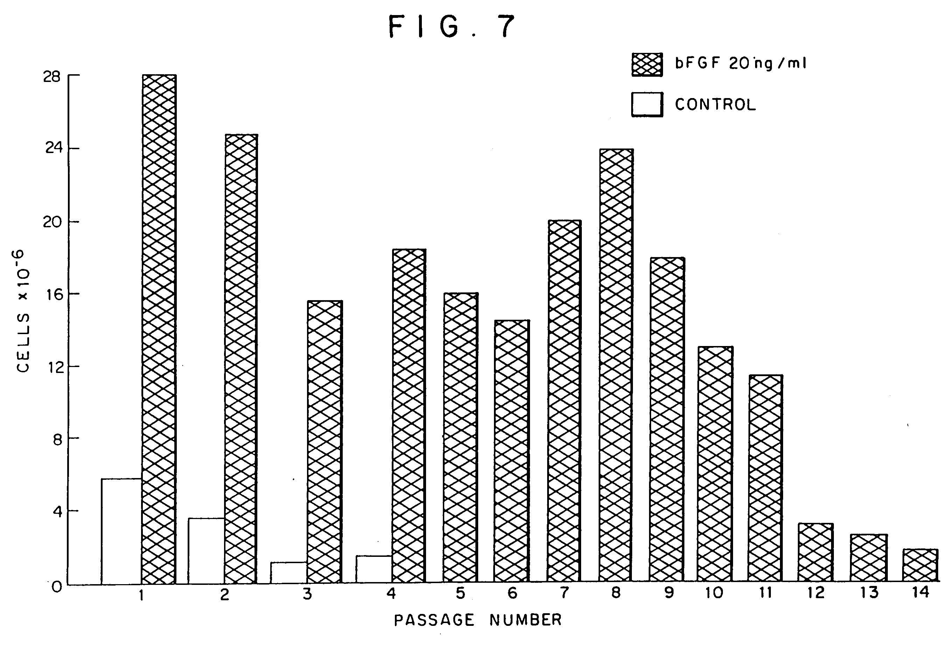

**Figure 7** is a bar graph comparing the density of cells cultured in the presence of bFGF versus control cells. Bone marrow stromal cells were seeded at 2 x 10⁷ buffy coat cells/75 cm² flask in the absence and presence of 20 ng/ml bFGF. At 10-12 day intervals, the cell number on each set of flasks was determined, and the cells were passaged at 1.3 x 10⁶/75 cm² flask.

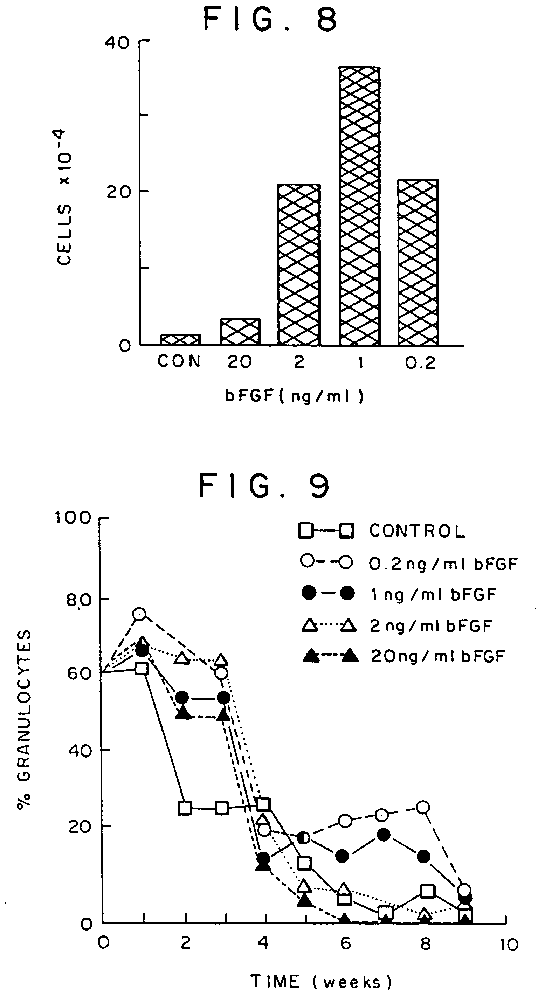

**Figure 8** is a bar graph showing enhancement in the generation of hemopoietic progenitors by bFGF. Human bone marrow cells were seeded at 2 x 10⁷ buffy coat cells/75 cm² flask in the absence and presence of 20, 2, 1, and 0.2 ng/ml bFGF. An adherent stromal cell layer containing foci of hemopoietic progenitor cells was established. The number of cells released into the culture medium was determined.

**Figure 9** is a graph showing release of granulocytes into culture medium under the influence of bFGF. Human bone marrow was seeded at 2 x 10⁷ buffy coat cells/75 cm² flask in the absence and presence of 20, 2, 1, and 0.2 ng/ml bFGF. At various intervals, cells in the supernatant were harvested and slides were prepared. A differential cell count identified the percentage of different cell types present in the specimens.

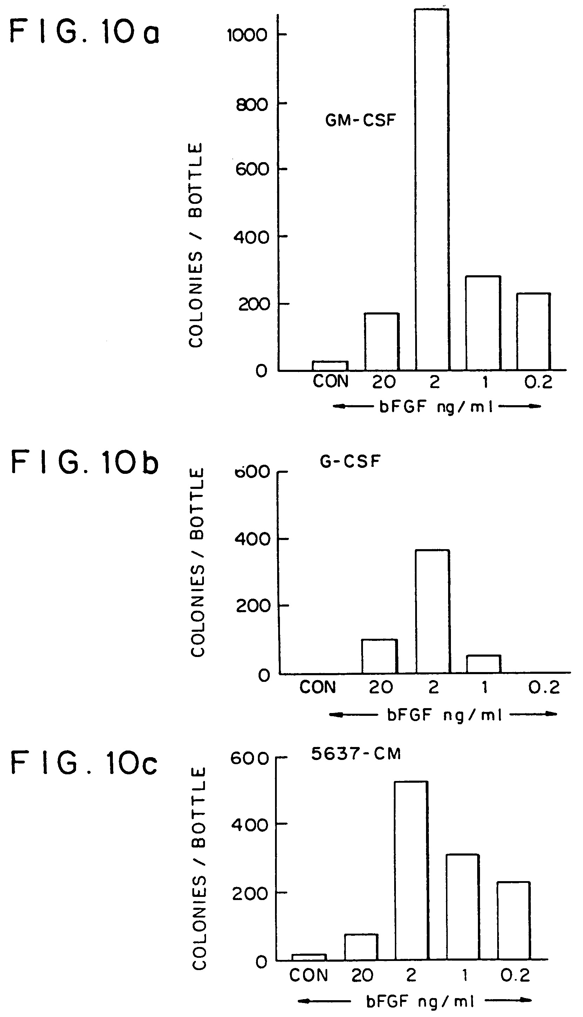

**Figure 10** is a set of bar graphs showing concentration-dependent stimulation of progenitor cell responses to CSFs by bFGF. Human bone marrow was seeded at 2 x 10⁷ buffy coat cells/75 cm² flask in the absence and presence of 20, 2, 1, and 0.2 ng/ml bFGF. At various intervals, cells in the supernatant were harvested and seeded in soft agar in the absence or presence of one of several CSFs (GM-CSF, G-CSF, and 5637-CM) which stimulate the growth of cells of myeloid origin.

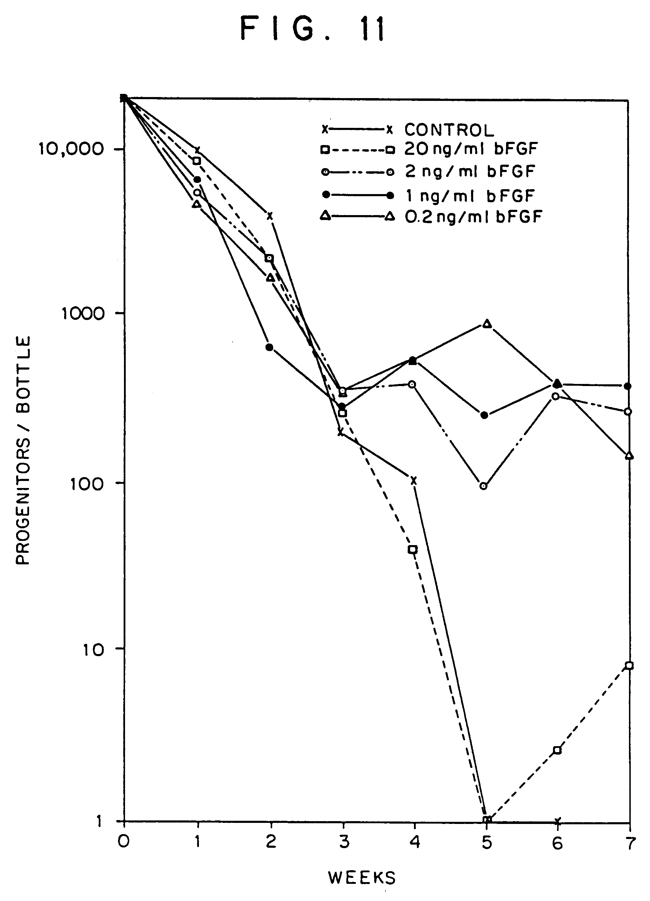

**Figure 11** is a graph illustrating that low concentrations of bFGF increase the progenitor cell population in the medium. Human bone marrow was seeded at 2 x 10⁷ buffy coat cells/75 cm² flask in the absence and presence of 20, 2, 1 and 0.2 ng/ml bFGF. At weekly intervals cells in the supernatant were harvested and seeded in soft agar in the presence of GM-CSF and colonies were counted after 14 days. Similar results were obtained after 7 days of culture and with G-CSF or 5637-CM.

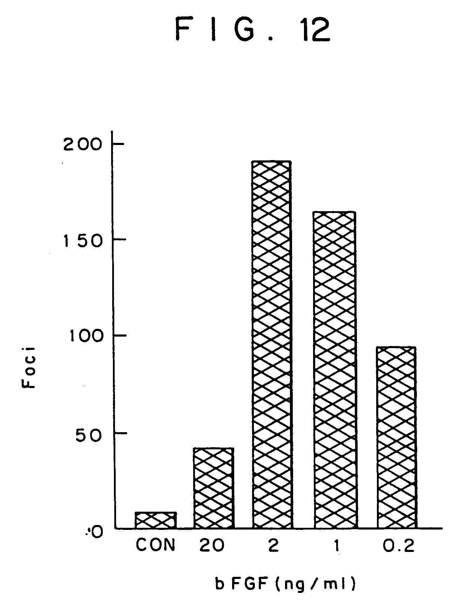

**Figure 12** is a bar graph showing establishment of an adherent stromal cell layer containing foci of hemopoietic progenitor cells in the presence of bFGF. Human bone marrow cells were seeded at 2 x 10⁷ buffy coat cells/75 cm² flask in the absence and presence of 20, 2, 1, and 0.2 ng/ml bFGF. An adherent stromal cell layer containing foci of hemopoietic progenitor cells was established.

The inventors have discovered that bFGF, a growth factor with a multiplicity of activities described above, can stimulate growth of bone marrow stromal cultures, which are capable of supporting hemopoietic cell growth and differentiation. Furthermore, the inventors have discovered that myelopoiesis in bone marrow culture is stimulated by bFGF. Furthermore, bFGF potentiates the effects of colony stimulating factors.

By the term "hemopoiesis" is intended the production of cellular elements of the blood, and includes myelopoiesis, lymphopoiesis, and erythropoiesis.

The term "hemopoietic progenitor cell" refers to a cell in any of the blood cell lineages (myeloid, erythroid, lymphoid, megakaryocytoid) which is a precursor of the mature blood cell. The term as used herein is intended to include very early precursors, such as pluripotent stem cells which are both self-renewing and give rise to all lineages, as well as committed stem cells which may have more limited self-renewal capacity than pluripotent stem cells, and are committed to a particular cell lineage. In particular, myeloid progenitor cells which are thought to be derived from the committed stem cells of the myeloid lineage, are the targets of the methods of the present invention. Generally, in mammals, the presence and proliferative activity of hemopoietic progenitor cells is limited to the bone marrow. However, to the extent that extramedullary hemopoiesis occurs, it is intended to be within the scope of the present invention.

Basic FGF stimulates the growth of bone marrow stromal cells, and greatly accelerates the formation of a primary stromal cell layer following culture of freshly harvested bone marrow cells. In the presence of bFGF, stromal cells attain high densities, lose their contact inhibition, and grow in multilayered sheets. The term "stromal cell" refers to a heterogeneous population of cells present in the bone marrow which grows in culture in the form of an adherent layer. Stromal cells include cells derived from the bone marrow which are referred to as adipocytes, preadipocytes, fibroblasts, fibroblast colony forming units (CFU-F), reticular cells and macrophages. Cloned stromal cell lines are also contemplated within the scope of the present invention. The state of the art of bone marrow stromal cells is reviewed by Dorshkind (Ann. Rev. Immunol. 8:111-137 (1990)), which is hereby incorporated by reference.

Heparan sulfate is known to be a component of the extracellular matrix which influences hemopoiesis. Growth factors such as colony stimulating factors have been shown to bind to heparin, serving as a possible mechanism by which stromal cells affect hemopoiesis (Roberts, R. et al., Nature 332:376-378 (1988)).

Heparin greatly potentiates the stimulatory effect of low concentrations of bFGF (Saksela, O. et al., J. Cell Biol. 107: 743-751 (1988); Ulrich, S. et al. Biochem. Biophys. Res. Commun. 137: 1205-1213 (1986); see Example below). In addition to heparin, the use of a heparin fragment or a synthetic heparin substitute is intended within the scope of the present invention. Heparin preparations are nonuniform and heterogeneous in composition, molecular size, structure, position of substituents (N-sulfate, O-sulfate, and glucuronic acid), and sequence (Goldgaber, et al., Science 235:877 (1987); Tanzi et al., Science 235:881 (1987); Robakis, N.K et al., Proc. Natl. Acad. Sci. USA 84:4190 (1987)). This heterogeneity is thought to be responsible for various effects observed.

Heparin can be modified, or heparin fragments synthesized by methods known in the art (Choay, J.et al., Biochem. Biophys. Res. Comm. 116:492 (1983); van Boeckel, C.A.A. et al., Tetrahedron Lett. 29:803 (1988), both of which references are hereby incorporated by reference).

Preferred heparin fragments include a hexasaccharide or a pentasaccharide fragment. Preferred synthetic heparin substitutes comprise cyclodextrins of six or eight glucopyranose units, such as, for example, β-cyclodextrin tetradecasulfate.

Cyclodextrins are naturally occurring cyclic nonreducing, water-soluble oligosaccharides built up from six to eight glucopyranose units (Bender, M.L. et al., Cyclodextrin Chemistry, Springer Verlag, Berlin, (1978); Saenger, W., Angew. Chem. Int. Ed. Engl. 91:344 (1980); Saenger, W., In: Inclusion Compounds (Atwood, J.L. et al., eds.), Academic Press, New York, 1984, vol. 2, pp. 232-259).

According to the present invention, bFGF is for administration to subjects to stimulate hemopoiesis (for example, in bone marrow transplant recipients) or to increase the number of progenitor cells (for example, in bone marrow donors).

The bFGF may be for administration by any means that achieve its intended purpose. For example, administration may be by various parenteral routes such as subcutaneous, intravenous, intradermal, intramuscular, intraperitoneal, intranasal, transdermal, or buccal routes. Alternatively, or concurrently, administration may be by the oral route. Parenteral administration can be by bolus injection or by gradual perfusion over time.

It is understood that the dosage of bFGF or of additional factors such as CSFs (see below) for administration in vivo or in vitro will be dependent upon the age, sex, health, and weight of the recipient, kind of concurrent treatment, if any, frequency of treatment, and the nature of the effect desired. The ranges of effective doses provided below are not intended to limit the inventors and represent preferred dose ranges. However, the most preferred dosage will be tailored to the individual subject, as is understood and determinable by one of skill in the art.

Preparations for parenteral administration include sterile aqueous or non-aqueous solutions, suspensions, and emulsions, which may contain auxiliary agents or excipients which are known in the art. Pharmaceutical compositions such as tablets and capsules can also be prepared according to routine method.

When used to promote growth of bone marrow either in bone marrow donors or in recipients, the bFGF is used for administration in an effective amount, preferably in the range of from about 0.1 µg/kg body weight to about 20 mg/kg body weight.

When used in vitro to promote bone marrow stromal cell or progenitor cell growth, the bFGF is preferably added to the medium to achieve a final concentration of about 0.01 to 10 µg\L.

Basic FGF is also used to enhance the production of granulocytes following chemotherapy for malignant diseases. Preferably, bFGF is for administration at a dose from about 0.1 µg to about 20 µg/kg body weight to protect a patient from infections which may result from the myelosuppressive effects of chemotherapy or from the other causes of myelosuppression known the art, which were discussed above.

To enhance the production of bone marrow cells and thereby promote hemopoietic reconstitution of a patient undergoing a bone marrow transplant, bFGF or a functional derivative thereof (defined below) is for administration in dosages preferably ranging from about 0.1 µg/kg body weight to about 20 mg/kg body weight of each agent. The bFGF is preferably for administration up to one week prior to the patient's receiving the bone marrow, which administration significantly shortens the "window" prior to the establishment of the graft.

bFGF or a functional derivative thereof in combination with one or more colony stimulating factors, preferably in dosages ranging from about 0.1 µg/kg body weight to about 20 mg/kg body weight of each agent is for administration to a patient undergoing a bone marrow transplant for enhancing the production of bone marrow cells. The mixture of bFGF and CSF is preferably for administration up to one week prior to the patient's receiving of the bone marrow transplant, which administration significantly shortens the "window" prior to the establishment of the graft during which time the recipient is myelosuppressed and particularly susceptible to infections.

As used herein, the term "colony stimulating factor" (CSF) is directed to any of the CSFs and related cytokines and lymphokines known in the art which stimulate aspects of hemopoiesis. Such factors include, but are not limited to, GM-CSF, G-CSF, M-CSF, IL-3, IL-4, and IL-6. For a discussion of the CSFs and their role in hemopoiesis, see Nienhuis, A., New Eng. J. Med., 318:916-918 (1988); Brandt et al. (supra), Cosman, D., Immunol. Today 9:97-98 (1988); and Dorshkind (supra).

The methods of the present invention contemplate the use of one or more than one CSF in combination with bFGF. One of ordinary skill in the art can readily determine which CSF or combination of CSFs to use, depending on the particular effect or effects desired.

According to the present invention, a bone marrow donor is given an effective amount of bFGF or a functional derivative thereof, prefereably from about 0.1 µg/kg body weight up to about 20 mg/kg body weight. This treatment is initiated at a time prior to obtaining the bone marrow sufficient for the progenitor cell number in the bone marrow to increase. A preferable time of treatment is for a period up to 2 weeks prior to obtaining the bone marrow. By enhancing the production of progenitor cells in the donor's bone marrow, this treatment is designed to enhance the efficiency of the bone marrow graft.

Where a suitable bone marrow donor is not available, the patient's own bone marrow cells are cultured in the presence of bFGF or a functional derivative thereof to provide stromal cell "feeder" layers for subsequent generation of primitive progenitor (stem) cells to be used for implantation into the patient. Methods for culture of stromal cells are described in Dexter, T.M. et al., Long-Term Bone Marrow Culture, ed. Wright, D.B. et al., Liss, New York, p. 441 (1984) and Dorshkind, K. et al. J. Immunol. Meth. 89:37-47 (1986), which are hereby incorporated by reference. bFGF may be added to stromal cell cultures as described in the Example, below. Alternatively, bFGF or a functional derivative thereof can be added directly to whole bone marrow cultures to increase the numbers of progenitor cells in a population to be used for transplantation.

Excessive bFGF production in the bone marrow may be involved in the etiology of myelofibrosis. Antibodies to bFGF or antagonists to the FGF receptor may be of use in treating individuals with myelofibrosis or related disease. Suramin may also be useful in treating myelofibrosis. This compound binds many growth factors including bFGF and has recently been used to treat cancer patients (Rocha, R.V. et al., Cancer Cells 2:105-115 (1990)).

The preferred animal subject of the present invention is a mammal. By the term "mammal" is meant an individual belonging to the class Mammalia. The invention is particularly useful in the treatment of human subjects, although it is intended for veterinary uses as well.

In addition to bFGF, the present invention is intended to encompass other fibroblast growth factors, and functional derivatives thereof having similar bioactivity for all the uses described herein. Also intended are all active forms of FGF derived from the FGF transcript, all muteins with FGF activity and all molecules which may interact with the FGF receptor. Other FGFs known in the art include acidic FGF, the hst/K-fgf gene product, FGF-5, and int-2 (see Rifkin, D.B. et al., supra).

Also intended within the scope of the present invention are the alternate molecular forms of bFGF, such as those having molecular weights of 17.8, 22.5, 23.1, and 24.2 kDa. The 17.8 kDa bFGF protein is translationally initiated at the previously predicted methionine (AUG) codon, whereas, the 22.5-, 23.1-, and 24.2-kDa proteins initiate at unusual non-AUG codons. The higher molecular weight forms are colinear N-terminal extensions of the 18 kDa bFGF (Florkiewicz et al., Proc. Natl. Acad. Sci. USA 86: 3978-3981 (1989), which reference is hereby incorporated by reference).

As alternatives to recombinant or purified intact bFGF, functional derivatives of the bFGF molecule may be used. See, for example, Arakawa et al., European Patent Publication EP320148, which describes recombinant bFGF analogs possessing part or all of the primary structural conformations and the biological properties of human bFGF. In these analogs, at least one of the cysteine residues of the naturally occurring basic fibroblast growth factor is replaced with a different amino acid residue.

Another form of bFGF which can be used according to the present invention is disclosed in Senoo et al. (European Patent Publication EP281822). The disclosed mutein includes modified, microbiologically-produced bioactive proteins which have the same activities as the naturally-occurring compound but are not capable of forming intermolecular bridges and intramolecular linkages via cysteine residues or others residues which result in the formation of undesirable tertiary structure (e.g., conformations which lower the protein activity).

By "functional derivative" is meant a "fragment," "variant," "analog," or "chemical derivative" of FGF, preferably by bFGF, which terms are defined below. A functional derivative retains at least a portion of the function of the bFGF which permits its utility in accordance with the present invention.

A "fragment" of bFGF refers to any subset of the molecule, that is, a shorter peptide.

A "variant" of bFGF refers to a molecule substantially similar to either the entire peptide or a fragment thereof. Variant peptides may be conveniently prepared by direct chemical synthesis of the variant peptide, using methods well-known in the art.

Alternatively, amino acid sequence variants of the peptide can be prepared by mutations in the DNA which encodes the synthesized peptide. Such variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame and preferably will not create complementary regions that could produce secondary mRNA structure (see European Patent Publication No. EP 75,444).

At the genetic level, these variants ordinarily are prepared by site-directed mutagenesis (as exemplified by Adelman et al., DNA 2:183 (1983)) of nucleotides in the DNA encoding the peptide molecule, thereby producing DNA encoding the variant, and thereafter expressing the DNA in recombinant cell culture. The variants typically exhibit the same qualitative biological activity as the nonvariant peptide.

An "analog" of bFGF refers to a non-natural molecule substantially similar to either the entire molecule or a fragment thereof.

A "chemical derivative" of bFGF contains additional chemical moieties not normally a part of the peptide. Covalent modifications of the peptide are included within the scope of this invention. Such modifications may be introduced into the molecule by reacting targeted amino acid residues of the peptide with an organic derivatizing agent that is capable of reacting with selected side chains or terminal residues.

Cysteinyl residues most commonly are reacted with alpha-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, alpha-bromo-beta-(5-imidazoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Parabromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing alpha-amino-containing residues include imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues per se has been studied extensively, with particular interest in introducing spectral labels into tyrosyl residues by reaction with aromatic diazonium compounds or tetranitromethane. Most commonly, N-acetylimidazol and tetranitromethane are used to form O-acetyl tyrosyl species and 3-nitro derivatives, respectively.

Carboxyl side groups (aspartyl or glutamyl) are selectively modified by reaction with carbodiimides (R'-N-C-N-R') such as 1-cyclohexyl-3-(2-morpholinyl-(4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Derivatization with bifunctional agents is useful for cross-linking the peptide to a water-insoluble support matrix or to other macromolecular carriers. Commonly used cross-linking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis-(succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Patent Nos. 3,969,287; 3,691,016; 4,195,128; 4,247,642; 4,229,537; and 4,330,440 are employed for protein immobilization.

Other modifications include hydroxylation of proline and lysine, phosphorylation of hydroxyl groups of seryl or threonyl residues, methylation of the alpha-amino groups of lysine, arginine, and histidine side chains (T.E. Creighton, Proteins: Structure and Molecule Properties, W.H. Freeman & Co., San Francisco, pp. 79-86 (1983)), acetylation of the N-terminal amine, and, in some instances, amidation of the C-terminal carboxyl groups.

Such derivatized moieties may improve the solubility, absorption, biological half life, and the like. The moieties may alternatively eliminate or attenuate any undesirable side effect of the protein and the like. Moieties capable of mediating such effects are disclosed, for example, in Remington's Pharmaceutical Sciences, 16th ed., Mack Publishing Co., Easton, PA (1980)

The following example is intended to be illustrative, but not to limit, the invention.

### EXAMPLE

### LONG TERM CULTURE OF HUMAN BONE MARROW STROMAL CELLS IN THE PRESENCE OF bFGF

The effect of bFGF on human bone marrow stromal cells was examined as a means for circumventing the difficulty of obtaining sufficient numbers of such cells.

### A. MATERIALS AND METHODS

Recombinant human bFGF was obtained from Synergen Inc. (Boulder, CO). Trypsin, heparin, and hydrocortisone were obtained from Sigma Chemical Co. (St. Louis, MO). The alpha minimal essential medium (αMEM) was obtained from Flow Laboratories (McLean, VA). Fetal calf serum was obtained from Armour Pharmaceutical CO (Kankakee, IL), and horse serum, from GIBCO (Grand Island, NY).

Human bone marrow stromal cells were obtained from healthy adult volunteers who had given informed consent. Buffy coat cells were seeded at a concentration of 3 x 10⁶/ml in "stromal medium" which consisted of αMEM containing 12.5% fetal calf serum, 12.5% horse serum, 10⁻⁶M hydrocortisone, 10⁻⁴M 2-mercaptoethanol, 1.6 mM glutamine and antibiotics. A layer of adherent cells was obtained within 2-3 weeks. Nonadherent cells were removed by extensive washing, and the adherent cell layer was passaged by first treating with 0.25% trypsin containing 0.02% EDTA to dislodge the cells. bFGF was added at the indicated concentrations. Fresh "stromal medium" was added at 48 hr intervals when testing the effects of bFGF on the growth of bone marrow stromal cells.

### B. RESULTS

### 1. Morphology

The effects of bFGF on the morphology of bone marrow stromal cells in culture were striking, as shown in Figure 1. The elongated fibroblastic morphology of stromal cells was altered by bFGF so that cells had a more compact morphology (Figures 1a and 1b). bFGF also altered the appearance of confluent cultures of cells. In the presence of bFGF, the cells grow to high density in multi-layers, while their counterparts, maintained in the absence of bFGF, were contact inhibited at a low cell density (Figures 1c and 1d).

### 2. Growth

The addition of bFGF increased the density of the primary stromal cell layer. Primary marrow buffy coat cells were seeded in "stromal medium" at 3 x 10⁶ cells/ml per 35 mm dish in the presence of 0. 0.2, 2, or 20 ng/ml bFGF and the cell number on duplicate dishes was determined 15 days later. As shown in Table 1, a 5-fold increase in cell density was noted when cells were cultured in the presence of 20 ng/ml bFGF; 0.2 ng bFGF/ml approximately doubled the cell density.

**Table 1**

| **The Effect of bFGF on the Growth of Primary Bone Marrow Stromal Cells** | |
|---|---|
| bFGF (ng/ml) | Cells/dish x 10⁻⁴ |
| 0 | 33.1 |
| 0.2 | 64.0 |
| 2. | 107.5 |
| 20 | 156.5 |
| Primary bone marrow buffy coat cells were seeded in stromal medium at 3 x10⁶ cells per 35 mm petri dish in the absence or presence of the indicated concentrations of bFGF. Cell numbers were determined on duplicate dishes after 15 days. | |

Culturing stromal cells continuously in the presence of bFGF delayed their senescence considerably. Stromal cells had a limited proliferative potential and senesced after approximately 2 generations, whereas cells cultured continuously in the presence of bFGF survived to approximately 26 generations, as shown in Table 2.

**Table 2**

| Effect on bFGF on the Senescence of Bone Marrow Stromal Cells | | |
|---|---|---|
| Experiment | Cell Generation | |
| | Control | bFGF Treated |
| 1 | ND^{a} | 29 |
| 2 | 3.1 | 21.8 |
| 3 | 0.8 | 16.8 |
| 4 | 1.4 | 39.5 |
| 5 | 1.8 | 24.7 |
| 6 | 2.2 | ND^{a} |
| Bone marrow was removed from 6 healthy volunteers and cultured in the absence and continuous presence of 20 ng/ml bFGF. At 10-13 day intervals the monolayers were removed with trypsin and 1.3 x 10⁶ cells were added to 75 cm² flasks. This process was continued until cell growth ceased. | | |

| | | |
|---|---|---|
| ^{a} - Not determined. | | |

Stimulatory effects on growth were also noted when stromal cell layers that had been passaged in culture were subsequently exposed to bFGF. Figure 2 illustrates the effect of varying concentrations of bFGF on stromal cells that had been passaged 3 times prior to its addition. Dishes treated with 20 ng/ml bFGF for 9 days contained 10 times the number of cells as found in untreated cultures. Low concentrations of bFGF (0.2 ng/ml) also significantly enhanced cell growth.

Stromal cells had a very limited proliferative potential when cultured in RPMI-1640 medium supplemented with 10% fetal calf serum, as shown in Figure 3. The addition of bFGF at 20 ng/ml to this medium greatly increased the proliferative potential of stromal cells; for example after 9 days of culture, with bFGF, a 12-fold increase in cell number was noted. Cells cultured in the presence of 20 ng/ml bFGF grow at a faster rate than those in "stromal medium" without bFGF. However, cells cultured in RPMI-1640 + bFGF did not reach as high a final density as cells cultured in "stromal medium" + bFGF.

Heparin potentiated growth stimulation by bFGF. The growth rate of cells cultured in the presence of 2 ng/ml bFGF and 20 µg/ml heparin was substantially greater than the growth rate of cells cultured in the presence of bFGF alone, and was somewhat greater than the growth rate of cells cultured in the presence of 20 ng/ml bFGF, as shown in Figure 4. Heparin alone, at 20 µg/ml, had little effect on cell growth.

The effects of bFGF on stromal cell growth were fully reversible. Cells that were cultured in the presence of 20 ng/ml bFGF for 2 passages over 5 weeks had the same rate of growth as untreated cells if the bFGF was removed prior to trypsinization and passaging of the monolayer, as shown in Figure 5. In contrast, significant stimulatory effects on growth were noted when bFGF was added to stromal cells for brief periods of time.

Even brief exposure to bFGF stimulates cell growth (Figure 6). A 3-fold increase in cell number was noted when the bFGF was added to cells for 24 hours before being removed and replaced with regular stromal medium.

Cells cultured in the presence of bFGF reached a much higher density than cells in control medium (Figure 7). Also, cells cultured in the presence of bFGF could be passaged many more times before senescence occurred. This occurred after approximately 26 generations, as compared to about 2 generations for cells cultured without bFGF. Basic FGF can therefore be used to generate large numbers of human bone marrow stromal cells, which has not previously been possible.

It should be noted that bFGF increased the number of hemopoietic cells released into the culture medium (Figure 8). Basic FGF, therefore, under these conditions, significantly stimulates the generation of hemopoietic progenitor cells in conjunction with a stromal cell layer.

The addition of low concentrations of bFGF to cell cultures resulted in an increased percentage of granulocytes shed into the medium (Figure 9). This experiment demonstrates that bFGF enhances the production of cells of the myeloid lineage. The enhanced production of granulocytes following bone marrow transplantation or following chemotherapy for malignant disease would protect the patient against infections.

bFGF at all concentrations tested stimulated the generation of progenitor cells able to respond to CSFs in vitro (Figures 10 and 11). GM-CSF, G-CSF, and 5637-conditioned medium (CM) all stimulated the growth of myeloid progenitors. GM-CSF acts on more primitive progenitor cells than G-CSF, and 5637-CM contains a mixture of different cytokines. The progenitors present in the medium of cells cultured with bFGF were therefore of myeloid origin.

The presence of bFGF in culture increased the number of progenitor cell-containing foci in the stromal cell layer (Figure 12). bFGF promoted the development of foci of progenitor cells in the adherent stromal layer and enhanced hemopoiesis in conjunction with the stromal cell layer.

### C. DISCUSSION

Basic FGF has previously been shown to be a potent mitogen for many mesoderm-derived cells and to delay senescence in certain cultured cells (Gospodarowicz et al., in vitro 14:85-118 (1978); Gospodarowicz et al., In Ford R.J. and Maizel A.L. (eds), Mediators in Cell Growth and Differentiation, Raven Press, New York, 1985, pp 109-134; Gospodarowicz et al., Endocr. Rev. 8:95-114 (1987)).

The present inventors have shown that at concentrations, as low as 0.2 ng/ml, bFGF is a potent mitogen for bone marrow stromal cells. The bFGF also enhances the final density attained by bone marrow stromal cell cultures, resulting in the cells forming dense multilayered sheets and having a transformed morphology. These effects are fully reversible. After exposure to bFGF for prolonged periods of time, once the bFGF is removed from the medium and the cells are trypsinized and passaged, the cell growth rate reverts to one comparable to that of untreated cells.

It has been demonstrated that bFGF binds to heparan sulfates present in cell matrices, and that interaction with heparin and heparan sulfate protects bFGF from proteolytic degradation (Sommer, A. et al., J. Cell. Physiol. 138:215-220 (1989); Saksela et al., J. Cell Biol. 107:743-751 (1988)). Matrix binding of bFGF may provide a reservoir of growth factor and may provide a continuous source of ligand for the specific high affinity receptors on the surfaces of cells (Moscatelli, D., J. Cell Biol. 107:753-759 (1988); Flaumenhaft, R. et al., J. Cell. Physiol. 140:75-81 (1989)). It has been shown herein that significant stimulation of growth follows a transient (4 hour) exposure of stromal cells to bFGF. Heparin potentiates the growth stimulating effect of bFGF, which may be because heparin protects bFGF from proteolytic degradation.

Bone marrow stromal cells are an essential component of the hemopoietic system and are known to be a source of the colony stimulating factors necessary for growth and differentiation of hemopoietic stem cells. Basic FGF has been isolated from preparations of bovine bone matrix (Hauschka, S.V. et al., J. Biol. Chem. 261:12665-12674 (1986)), and it may be an important factor required for the function and proliferation of the stromal cell component of the hemopoietic system.

The fact that bFGF, when added to stromal medium, permits the growth of large numbers of human stromal fibroblasts in vitro provides a more readily available source of these fibroblasts for investigating their response to a variety of cytokines relevant to bone marrow physiology.

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

## Claims

1. Use of a fibroblast growth factor or a functional derivative thereof for the preparation of a composition for stimulating proliferation of hemopoietic progenitor cells in vivo or in vitro.

2. Use of claim 1 wherein said fibroblast growth factor is selected from basic fibroblast growth factor, acidic fibroblast growth factor, the hst/K-fgf gene product, FGF-5 or int-2.

3. Use of claim 2, wherein said fibroblast growth factor is a basic fibroblast growth factor or a functional derivative thereof.

4. Use of claim 3, wherein said basic fibroblast growth factor is selected from the 17.5 kilodalton, the 22.5 kilodalton, the 23.1 kilodalton or the 24.2 kilodalton forms of said fibroblast growth factor.

5. Use of any one of claims 1 to 4, wherein the composition is a pharmaceutical composition.

6. A method for proliferating hemopoietic progenitor cells cultured in vitro, comprising culturing said hemopoietic progenitor cells in a culture medium containing a proliferating effective amount of a fibroblast growth factor or functional derivative thereof, for a time sufficient to permit proliferation of the hematopoietic progenitor cells.

7. The method of claim 6, wherein said fibroblast growth factor is selected from basic fibroblast growth factor, acidic fibroblast growth factor, the hst/K-fgf gene product, FGF-5 or int-2.

8. The method of claim 7, wherein said fibroblast growth factor is basic fibroblast growth factor or a functional derivative thereof.

9. The method of claim 8, wherein said basic fibroblast growth factor is selected from the 17.5 kilodalton, the 22.5 kilodalton, the 23.1 kilodalton or the 24.2 kilodalton forms of said fibroblast growth factor.

10. A pharmaceutical composition for accelerating the engraftment of transplanted hemopoietic bone marrow cells in a mammalian subject or for treating a mammalian subject having a disease associated with dysplasia or aplasia of a bone marrow cell lineage comprising a combination of a fibroblast growth factor or a functional derivative thereof and at least one colony stimulating factor.

11. A pharmaceutical composition for stimulating the proliferation of hemopoietic progenitor cells in the bone marrow of a prospective mammalian bone marrow donor in vivo, comprising a combination of a fibroblast growth factor or a functional derivative thereof and an effective amount of heparin or a heparin analog, said pharmaceutical composition being suitable for administration at a time prior to the harvesting of said bone marrow.

12. The pharmaceutical composition of claim 10 or 11, wherein said fibroblast growth factor is selected from basic fibroblast growth factor, acidic fibroblast growth factor, the hst/K-fgf gene product, FGF-5 or int-2.

13. The pharmaceutical composition of claim 12, wherein said fibroblast growth factor is basic fibroblast growth factor or a functional derivative thereof.

14. The pharmaceutical composition of claim 13, wherein said basic fibroblast growth factor is selected from the 17.5 kilodalton, the 22.5 kilodalton, the 23.1 kilodalton or the 24.2 kilodalton forms of said fibroblast growth factor.

15. The pharmaceutical composition of any one of claims 10 or 12 to 14, wherein said colony stimulating factor is selected from GM-CSF, G-CSF, M-CSF, Interleukin-3, Interleukin-4 or Interleukin-6.

16. Use of a fibroblast growth factor or a functional derivative thereof for the preparation of a pharmaceutical composition for stimulating the proliferation of hemopoietic progenitor cells in the bone marrow of a prospective mammalian bone marrow donor in vivo, said pharmaceutical composition being suitable for administration at a time prior to the harvesting of said bone marrow.

17. Use of claim 16, wherein said fibroblast growth factor is selected from basic fibroblast growth factor, acidic fibroblast growth factor, the hst/K-fgf gene product, FGF-5 or int-2.

18. Use of claim 17, wherein said fibroblast growth factor is basic fibroblast growth factor or a functional derivative thereof.

19. Use of claim 18, wherein said basic fibroblast growth factor is selected from the 17.5 kilodalton, the 22.5 kilodalton, the 23.1 kilodalton or the 24.2 kilodalton forms of said fibroblast growth factor.

20. Use of a fibroblast growth factor or a functional derivative thereof for the preparation of a pharmaceutical composition for treating a mammalian subject having a disease associated with dysplasia or aplasia of a bone marrow cell lineage.

21. Use of claim 20, wherein said fibroblast growth factor is selected from basic fibroblast growth factor, acidic fibroblast growth factor, the hst/K-fgf gene product, FGF-5 or int-2.

22. Use of claim 21, wherein said fibroblast growth factor is basic fibroblast growth factor or a functional derivative thereof.

23. Use of claim 22, wherein said basic fibroblast growth factor is selected from the 17.5 kilodalton, the 22.5 kilodalton, the 23.1 kilodalton, or the 24.2 kilodalton forms of said fibroblast growth factor.

24. Use of any one of claims 20 to 23, wherein the composition comprises in combination with said fibroblast growth factor or derivative thereof, at least one colony stimulating factor.

25. Use of claim 24, wherein said colony stimulating factor is selected from GM-CSF, G-CSF, M-CSF, Interleukin-3, Interleukin-4 or Interleukin-6.

26. The composition of any one of claims 10 to 15, wherein said cells are human cells.

27. The method or use of any one of claims 1 to 9 and 16 to 24 wherein said cells are human cells.

28. The pharmaceutical composition of any one of claims 10 to 15 for administration to human patients.

## Patentansprüche

1. Verwendung eines Fibroblasten-Wachstumsfaktors oder eines funktionellen Derivats davon zur Herstellung einer Zusammensetzung zur Stimulierung der Proliferation hämatopoetischer Vorläuferzellen in vivo oder in vitro.

2. Verwendung nach Anspruch 1, wobei der FibroblastenWachstumsfaktor basischer Fibroblasten-Wachstumsfaktor, saurer Fibroblasten-Wachstumsfaktor, das hst/K-fgf-Genprodukt, FGF-5 oder int-2 ist.

3. Verwendung nach Anspruch 2, wobei der Fibroblasten-Wachstumsfaktor basischer Fibroblasten-Wachstumsfaktor oder ein funktionelles Derivat davon ist.

4. Verwendung nach Anspruch 3, wobei der basische Fibroblasten-Wachstumsfaktor die 17,5 Kilodalton-Form, die 22,5 Kilodalton-Form, die 23,1 Kilodalton-Form oder die 24,2 Kilodalton-Form dieses Fibroblasten-Wachstumsfaktors ist.

5. Verwendung nach einem der Ansprüche 1 bis 4, wobei die Zusammensetzung ein Arzneimittel ist.

6. Verfahren zur Vermehrung in vitro gezüchteter hämatopoetischer Vorläuferzellen, wobei man die hämatopoetischen Vorläuferzellen für eine Zeit, die ausreichend ist, um die Vermehrung hämatopoetischer Vorläuferzellen zu erlauben, in einem Kulturmedium züchtet, das eine für die Proliferation wirksame Menge eines Fibroblasten-Wachstumsfaktors oder eines funktionellen Derivats davon enthält.

7. Verfahren nach Anspruch 6, wobei der Fibroblasten-Wachstumsfaktor basischer Fibroblasten-Wachstumsfaktor, saurer Fibroblasten-Wachstumsfaktor, das hst/K-fgf-Genprodukt, FGF-5 oder int-2 ist.

8. Verfahren nach Anspruch 7, wobei der Fibroblasten-Wachstumsfaktor basischer Fibroblasten-Wachstumsfaktor oder ein funktionelles Derivat davon ist.

9. Verfahren nach Anspruch 8, wobei der basische Fibroblasten-Wachstumsfaktor die 17,5 Kilodalton-Form, die 22,5 Kilodalton-Form, die 23,1 Kilodalton-Form oder die 24,2 Kilodalton-Form dieses Fibroblasten-Wachstumsfaktors ist.

10. Arzneimittel zur Beschleunigung der Verankerung von transplantierten hämatopoetischen Knochenmarkszellen in einem Säuger oder zur Behandlung eines Säugers, der eine Krankheit aufweist, die mit der Dysplasie oder Aplasie einer Knochenmarkszellinie assoziiert ist, das eine Kombination eines Fibroblasten-Wachstumsfaktors oder eines funktionellen Derivats davon und mindestens eines Kolonie-stimulierenden Faktors umfaßt.

11. Arzneimittel zur Stimulierung der in vivo-Proliferation hämatopoetischer Vorläuferzellen im Knochenmark eines potentiellen Knochenmarkspenders, der ein Säuger ist, das eine Kombination eines Fibroblasten-Wachstumsfaktors oder eines funktionellen Derivats davon und einer wirksamen Menge Heparin oder eines Heparinanalogs umfaßt, und zur Verabreichung zu einem Zeitpunkt vor Knochenmarksentnahme geeignet ist.

12. Arzneimittel nach Anspruch 10 oder 11, wobei der Fibroblasten-Wachstumsfaktor basischer Fibroblasten-Wachstumsfaktor, saurer Fibroblasten-Wachstumsfaktor, das hst/K-fgf-Genprodukt, FGF-5 oder int-2 ist.

13. Arzneimittel nach Anspruch 12, wobei der Fibroblasten-Wachstumsfaktor basischer Fibroblasten-Wachstumsfaktor oder ein funktionelles Derivat davon ist.

14. Arzneimittel nach Anspruch 13, wobei der basische Fibroblasten-Wachstumsfaktor die 17,5 Kilodalton-Form, die 22,5 Kilodalton-Form, die 23,1 Kilodalton-Form oder die 24,2 Kilodalton-Form dieses Fibroblasten-Wachstumsfaktors ist.

15. Arzneimittel nach einem der Ansprüche 10 oder 12 bis 14, wobei der Kolonie-stimulierende Faktor GM-CSF, G-CSF, M-CSF, Interleukin-3, Interleukin-4 oder Interleukin-6 ist.

16. Verwendung eines Fibroblasten-Wachstumsfaktors oder eines funktionellen Derivats davon zur Herstellung eines Arzneimittels zur Stimulierung der in vivo-Proliferation hämatopoetischer Vorläuferzellen im Knochenmark eines potentiellen Knochenmarkspenders, der ein Säuger ist, das zur Verabreichung zu einem Zeitpunkt vor Knochenmarksentnahme geeignet ist.

17. Verwendung nach Anspruch 16, wobei der FibroblastenWachstumsfaktor basischer Fibroblasten-Wachstumsfaktor, saurer Fibroblasten-Wachstumsfaktor, das hst/K-fgf-Genprodukt, FGF-5 oder int-2 ist.

18. Verwendung nach Anspruch 17, wobei der Fibroblasten-Wachstumsfaktor basischer Fibroblasten-Wachstumsfaktor oder ein funktionelles Derivat davon ist.

19. Verwendung nach Anspruch 18, wobei der basische Fibroblasten-Wachstumsfaktor die 17,5 Kilodalton-Form, die 22,5 Kilodalton-Form, die 23,1 Kilodalton-Form oder die 24,2 Kilodalton-Form dieses Fibroblasten-Wachstumsfaktors ist.

20. Verwendung eines Fibroblasten-Wachstumsfaktors oder eines funktionellen Derivats davon zur Herstellung eines Arzneimittels zur Behandlung eines Säugers, der eine Krankheit aufweist, die mit der Dysplasie oder Aplasie einer Knochenmarkszellinie assoziiert ist.

21. Verwendung nach Anspruch 20, wobei der Fibroblasten-Wachstumsfaktor basischer Fibroblasten-Wachstumsfaktor, saurer Fibroblasten-Wachstumsfaktor, das hst/K-fgf-Genprodukt, FGF-5 oder int-2 ist.

22. Verwendung nach Anspruch 21, wobei der Fibroblasten-Wachstumsfaktor basischer Fibroblasten-Wachstumsfaktor oder ein funktionelles Derivat davon ist.

23. Verwendung nach Anspruch 22, wobei der basische Fibroblasten-Wachstumsfaktor die 17,5 Kilodalton-Form, die 22,5 Kilodalton-Form, die 23,1 Kilodalton-Form oder die 24,2 Kilodalton-Form dieses Fibroblasten-Wachstumsfaktors ist.

24. Verwendung nach einem der Ansprüche 20 bis 23, wobei das Arzneimittel in Kombination mit dem Fibroblasten-Wachstumsfaktor oder einem Derivat davon mindestens einen Kolonie-stimulierenden Faktor umfaßt.

25. Verwendung nach Anspruch 24, wobei der Kolonie-stimulierende Faktor GM-CSF, G-CSF, M-CSF, Interleukin-3, Interleukin-4 oder Interleukin-6 ist.

26. Arzneimittel nach einem der Ansprüche 10 bis 15, wobei die Zellen menschliche Zellen sind.

27. Verfahren oder Verwendung nach einem der Ansprüche 1 bis 9 und 16 bis 24, wobei die Zellen menschliche Zellen sind.

28. Arzneimittel nach einem der Ansprüche 10 bis 15 zur Verabreichung bei menschlichen Patienten.

## Revendications

1. Utilisation d'un facteur de croissance des fibroblastes ou d'un dérivé fonctionnel de celui-ci pour la préparation d'une composition pour stimuler la prolifération de cellules précurseurs hématopoïétiques *in vivo* ou *in vitro*.

2. Utilisation selon la revendication 1, dans laquelle ledit facteur de croissance des fibroblastes est choisi parmi un facteur de croissance des fibroblastes basique, un facteur de croissance des fibroblastes acide, le produit génique hst/K-fgf, FGF-5 ou int-2.

3. Utilisation selon la revendication 2, dans laquelle ledit facteur de croissance des fibroblastes est un facteur de croissance des fibroblastes basique ou un dérivé fonctionnel de celui-ci.

4. Utilisation selon la revendication 3, dans laquelle ledit facteur de croissance des fibroblastes basique est choisi parmi les formes de 17,5 kilodaltons, de 22,5 kilodaltons, de 23,1 kilodaltons ou de 24,2 kilodaltons dudit facteur de croissance des fibroblastes.

5. Utilisation selon l'une quelconque des revendications 1 à 4, dans laquelle la composition est une composition pharmaceutique.

6. Procédé pour faire proliférer des cellules précurseurs hématopoïétiques cultivées *in vitro*, comprenant la culture desdites cellules précurseurs hématopoïétiques dans un milieu de culture contenant une quantité proliférative efficace d'un facteur de croissance des fibroblastes ou d'un dérivé fonctionnel de celui-ci, pendant un temps suffisant pour permettre la prolifération des cellules précurseurs hématopoïétiques.

7. Procédé selon la revendication 6, dans lequel ledit facteur de croissance des fibroblastes est choisi parmi un facteur de croissance des fibroblastes basique, un facteur de croissance des fibroblastes acide, le produit génique hst/K-fgf, FGF-5 ou int-2.

8. Procédé selon la revendication 7, dans lequel ledit facteur de croissance des fibroblastes est un facteur de croissance des fibroblastes basique ou un dérivé fonctionnel de celui-ci.

9. Procédé selon la revendication 8, dans lequel ledit facteur de croissance des fibroblastes basique est choisi parmi les formes de 17,5 kilodaltons, de 22,5 kilodaltons, de 23,1 kilodaltons ou de 24,2 kilodaltons dudit facteur de croissance des fibroblastes.

10. Composition pharmaceutique pour accélérer la prise de greffe de cellules de moelle osseuse hématopoïétiques transplantées dans un sujet mammifère ou pour traiter un sujet mammifère ayant une maladie associée avec une dysplasie ou une aplasie d'une lignée de cellules de moelle osseuse, comprenant une combinaison d'un facteur de croissance des fibroblastes ou d'un dérivé fonctionnel de celui-ci et d'au moins un facteur stimulateur de colonie.

11. Composition pharmaceutique pour stimuler la prolifération de cellules précurseurs hématopoïétiques dans la moelle osseuse d'un donneur potentiel de moelle osseuse mammifère *in vivo*, comprenant une combinaison d'un facteur de croissance des fibroblastes ou d'un dérivé fonctionnel de celui-ci et d'une quantité efficace d'héparine ou d'un analogue de l'héparine, ladite composition pharmaceutique étant appropriée pour l'administration à un temps avant la récolte de ladite moelle osseuse.

12. Composition pharmaceutique selon la revendication 10 ou 11, dans laquelle ledit facteur de croissance des fibroblastes est choisi parmi un facteur de croissance des fibroblastes basique, un facteur de croissance des fibroblastes acide, le produit génique hst/K-fgf, FGF-5 ou int-2.

13. Composition pharmaceutique selon la revendication 12, dans laquelle ledit facteur de croissance des fibroblastes est un facteur de croissance des fibroblastes basique ou un dérivé fonctionnel de celui-ci.

14. Composition pharmaceutique selon la revendication 13, dans laquelle ledit facteur de croissance des fibroblastes basique est choisi parmi les formes de 17,5 kilodaltons, de 22,5 kilodaltons, de 23,1 kilodaltons ou de 24,2 kilodaltons dudit facteur de croissance des fibroblastes.

15. Composition pharmaceutique selon l'une quelconque des revendications 10 ou 12 à 14, dans lequel ledit facteur stimulateur de colonie est choisi parmi GM-CSF, G-CSF, M-CSF, l'Interleukine-3, l'Interleukine-4 ou l'Interleukine-6.

16. Utilisation d'un facteur de croissance des fibroblastes ou d'un dérivé fonctionnel de celui-ci pour la préparation d'une composition pharmaceutique pour stimuler la prolifération de cellules précurseurs hématopoïétiques dans la moelle osseuse d'un donneur potentiel de moelle osseuse mammifère *in vivo*, ladite composition pharmaceutique étant appropriée pour l'administration à un temps avant la récolte de ladite moelle osseuse.

17. Utilisation selon la revendication 16, dans laquelle ledit facteur de croissance des fibroblastes est choisi parmi un facteur de croissance des fibroblastes basique, un facteur de croissance des fibroblastes acide, le produit génique hst/K-fgf, FGF-5 ou int-2.

18. Utilisation selon la revendication 17, dans laquelle ledit facteur de croissance des fibroblastes est un facteur de croissance des fibroblastes basique ou un dérivé fonctionnel de celui-ci.

19. Utilisation selon la revendication 18, dans laquelle ledit facteur de croissance des fibroblastes basique est choisi parmi les formes de 17,5 kilodaltons, de 22,5 kilodaltons, de 23,1 kilodaltons ou de 24,2 kilodaltons dudit facteur de croissance des fibroblastes.

20. Utilisation d'un facteur de croissance des fibroblastes ou d'un dérivé fonctionnel de celui-ci pour la préparation d'une composition pharmaceutique pour traiter un sujet mammifère ayant une maladie associée avec une dysplasie ou une aplasie d'une lignée de cellules de moelle osseuse.

21. Utilisation selon la revendication 20, dans laquelle ledit facteur de croissance des fibroblastes est choisi parmi un facteur de croissance des fibroblastes basique, un facteur de croissance des fibroblastes acide, le produit génique hst/K-fgf, FGF-5 ou int-2.

22. Utilisation selon la revendication 21, dans laquelle ledit facteur de croissance des fibroblastes est un facteur de croissance des fibroblastes basique ou un dérivé fonctionnel de celui-ci.

23. Utilisation selon la revendication 22, dans laquelle ledit facteur de croissance des fibroblastes basique est choisi parmi les formes de 17,5 kilodaltons, de 22,5 kilodaltons, de 23,1 kilodaltons ou de 24,2 kilodaltons dudit facteur de croissance des fibroblastes.

24. Utilisation selon l'une quelconque des revendications 20 à 23, dans laquelle la composition comprend, en combinaison avec ledit facteur de croissance des fibroblastes ou dérivé de celui-ci, au moins un facteur stimulateur de colonie.

25. Utilisation selon la revendication 24, dans laquelle ledit facteur stimulateur de colonie est choisi parmi GM-CSF, G-CSF, M-CSF, l'Interleukine-3, l'Interleukine-4 ou l'Interleukine-6.

26. Composition selon l'une quelconque des revendications 10 à 15, dans laquelle lesdites cellules sont des cellules humaines.

27. Procédé ou utilisation selon l'une quelconque des revendications 1 à 9 et 16 à 24, où lesdites cellules sont des cellules humaines.

28. Composition pharmaceutique selon l'une quelconque des revendications 10 à 15, pour l'administration à des patients humains.
